# EUROPEAN PATENT APPLICATION

(11) **EP 3 978 075 A1**
(43) Date of publication of application: **06.04.2022**
(21) Application number: 20819234.4
(22) Date of filing: 01.06.2020
(51) Int. Cl.: A61P 21/00, A61K 9/48, A23L 33/135, A61K 35/747

(54) **WALKING ABILITY IMPROVER**

(30) Priority: 03.06.2019 JP 2019103777
(71) Applicant: Asahi Group Holdings, Ltd., Tokyo 130-8602 (JP)
(72) Inventor: SAKATA, Shinji, Moriya-shi, Ibaraki 302-0106 (JP)
(74) Representative: Müller-Boré & Partner Patentanwälte PartG mbB
(86) International application number: PCT/JP2020/021637
(87) International publication number: WO 2020/246430

(57) **Abstract**

Provided are a walking ability improver for improving walking ability, and a health-related QOL improver for improving mental health. The walking ability improver contains a component derived from a lactic acid bacterium belonging to the genus *Lactobacillus.* The health-related QOL improver contains a component derived from a lactic acid bacterium belonging to the genus *Lactobacillus,* and improves mental health.

## Description

### TECHNICAL FIELD

The present invention relates to a walking ability improver.

### BACKGROUND ART

Walking ability refers to an ability to implement smooth walking movement and is considered to be related to the motor function and health-related QOL of the elderly. The walking ability is an index comprehensively reflecting functions related to motions of the heart, lungs, muscles, etc. Therefore, maintaining and improving the walking ability declining due to aging etc. contribute to an improvement in the health of the elderly. The walking ability can be rephrased as mobility ability, and the decrease thereof may cause a mental pain such as being unable to cross a pedestrian crossing, having difficulty changing trains at stations, and being unable to keep up with group activities such as travel. As a result, if the frequency of going out decreases as the walking ability declines, the person may withdraw, become weak, and fall into a state of requiring long-term care. Therefore, the improvement in walking ability leads to an improvement in motor function and health-related QOL.

In this regard, for example, Japanese Laid-Open Patent Publication No. 2013-47192 describes a physical activity promoter containing *Lactobacillus gasseri* as an active ingredient. Japanese Laid-Open Patent Publication No. 2018-199642 describes a muscle modifier containing a complex bacterium derived from a high-temperature fermentation product. Japanese Laid-Open Patent Publication No. 2016-216408 describes a muscle decomposition inhibitor containing a lactic acid bacterium strain that is *Lactobacillus curvatus* or *Lactobacillus amylovorus* etc. In the Yakult Bio-Science Foundation Annual Report 21: 114-121 (2013) "Mechanism of Maintaining Intestinal Function and Preventing Sarcopenia (disuse muscle atrophy)", it is described that *Lactobacillus casei shirota* has improved a process of recovery from muscle damage caused by snake venom.

### SUMMARY OF THE INVENTION

### PROBLEM TO BE SOLVED BY THE INVENTION

The physical activity promoting action in Japanese Laid-Open Patent Publication No. 2013-47192 means increases in amount of activity and muscle mass in a mouse, which is a concept different from the improvement in walking ability. The muscle modifying action described in Japanese Laid-Open Patent Publication No. 2018-199642 is an action causing a qualitative change in muscle fibers and is an action different from the improvement in walking ability. The muscle decomposition inhibitor described in Japanese Laid-Open Patent Publication No. 2016-216408 is not directly related to the improvement in walking ability although suppressing the decrease of skeletal muscle. The the Yakult Bio-Science Foundation Annual Report 21: 114-121 (2013) "Mechanism of Maintaining Intestinal Function and Preventing Sarcopenia (disuse muscle atrophy)" is a document related to the promotion of muscle synthesis and is not directly related to the improvement in walking ability. An object of an aspect of the present invention is to provide a walking ability improver improving a walking ability.

### MEANS FOR SOLVING PROBLEM

One aspect of the present invention provides a walking ability improver containing a component derived from a lactic acid bacterium belonging to the genus *Lactobacillus.* In one aspect, the walking ability improver may improve a walking speed. In one aspect, the walking ability improver may improve an amount of muscle activity. Another aspect provides a health-related QOL improver containing a component derived from a lactic acid bacterium belonging to the genus *Lactobacillus* and improving a mental health degree. In one aspect, the health-related QOL improver may improve vitality out of the mental health degree. In one aspect, the health-related QOL improver may improve mental health out of the mental health degree. In one aspect, the lactic acid bacterium may be *Lactobacillus curvatus.*

One aspect of the present invention provides a food or drink for improving a walking ability containing a component derived from a lactic acid bacterium belonging to the genus *Lactobacillus.* In one aspect, the food or drink may improve a walking speed. In one aspect, the food or drink may improve an amount of muscle activity. Another aspect provides a food or drink for improving health-related QOL, containing a component derived from a lactic acid bacterium belonging to the genus *Lactobacillus* and improving a mental health degree. In one aspect, the food or drink for improving health-related QOL may improve vitality out of the mental health degree. In one aspect, the food or drink for improving health-related quality of life may improve mental health out of the mental health degree. In one aspect, the lactic acid bacterium may be *Lactobacillus curvatus.*

### EFFECT OF THE INVENTION

An aspect of the present invention can provide the walking ability improver improving a walking ability.

### BRIEF DESCRIPTION OF DRAWINGS

Fig. 1A is a graph showing a transition of walking speed.
Fig. 1B is a graph showing a transition of cadence.
Fig. 1C is a graph showing a transition of stride.
Fig. 2 is a graph showing a transition of myoelectric potential.
Fig. 3A is a graph showing an evaluation result of a mental health degree of SF-36.
Fig. 3B is a graph showing evaluation results of vitality and mental health out of the mental health degree of SF-36.
Fig. 4 is a graph showing a transition of muscle mass.

### MODES FOR CARRYING OUT THE INVENTION

Embodiments of the present invention will now be described in detail. The embodiments described below exemplify a walking ability improver etc. for embodying the technical idea of the present invention, and the present invention is not limited to the walking ability improver etc. described below. As used herein, the term "improvement" means at least one of quantitative improvement and qualitative improvement in a measured value serving as an index.

The walking ability improver contains a component derived from a lactic acid bacterium belonging to the genus *Lactobacillus.* By ingesting the component derived from a lactic acid bacterium belonging to the genus *Lactobacillus,* walking ability is improved due to, for example, an improvement in amount of muscle activity and an improvement in walking speed, and as a result, maintenance/improvement in health-related QOL can be achieved. Therefore, the walking ability improver may be at least one selected from the group consisting of a walking speed improver, a muscle activity improver, and a health-related QOL improver.

The walking ability is considered to be related to factors such as speed, endurance, stability, and efficiency and can be evaluated by comfortable walking speed, for example. Therefore, the improvement in walking ability means, for example, an improvement in walking speed. The improvement in walking speed includes at least one of an improvement in cadence and an improvement in stride. The improvement in walking speed can be evaluated by, for example, measuring a lower limb load with a sheet-type foot pressure contact footprint measuring device, performing time/distance factor analysis during walking, and measuring cadence and stride. An improvement in at least one of cadence and stride may result from, for example, an improvement in amount of muscle activity via neural response. For example, an amount of muscle activity can be evaluated by measuring the myoelectric potential of the thigh during isometric knee extension exercise with a myoelectric potential meter.

Regarding quality of life (QOL), extension of healthy life expectancy and improvement in QOL have been described for the purpose of maintaining and improving national health in Health Japan 21 (see White Paper on Health, Labor and Welfare (18) 204-218) established by the Ministry of Health, Labor and Welfare. In the description, mental health is mentioned as an element of QOL. The mental health in this case means having a purpose of life, living enthusiastically, being active, etc. Since improvements in mental health as well as physical function is related to the improvement in QOL, these are collectively defined as health-related QOL. Therefore, the health-related QOL is a portion of the QOL directly affecting human health and includes a physical state, a psychological state, a social state, a spiritual state, a role function, overall well-being, etc. For the health-related QOL, see, for example, Japan Journal of Physical Education, Health and Sport Sciences 51: 103-115 (2006).

The health-related QOL is evaluated as a comprehensive scale (questionnaire). Medical Outcome Study Short Form 36 (SF-36) is used as a medical comprehensive scale. SF-36 is the most used health scale in the world and is a self-reported health state questionnaire. SF-36 is an evaluation method developed in US in the 1980s, and a Japanese version was created in 1992, and guidelines have been set. SF-36 is composed of 36 subordinate concepts, 8 intermediate concepts, and 2 superordinate concepts, and 36 questions are scored to evaluate the health condition. SF-36v2 is an improved version of SF-36.

The two superordinate concepts in the health-related QOL are "physical health degree" and "mental health degree". The "physical health degree" is composed of five intermediate concepts of "physical functioning", "role functioning (body)", "bodily pain", "general health perception", and "vitality". The "mental health degree" is composed of five intermediate concepts of "general health perception", "vitality", "social functioning", "role functioning (mental)", and "mental health". SF-36v2 can reflect age, gender, health status, etc. Generally, the score of SF-36v2 decreases due to aging, illness, etc. For details of SF-36v2, see, for example, SF-36v2 Japanese version manual (January 2015 version).

The "vitality" in health-related QOL means, for example, being full of vitality, being less tired, and being full of energy. The "mental health" means, for example, no longer feeling melancholy, being calm, happy, and peaceful, and not being depressed.

The health-related QOL improved by the walking ability improver may be either "physical health degree" or "mental health degree" and may include at least "mental health degree". The "mental health degree" improved by the walking ability improver may be any of "general health perception", "vitality", "social functioning", "role functioning (mental)", and "mental health" and may include at least one of "vitality" and "mental health", for example.

Components derived from lactic acid bacteria contained in the walking ability improver and the health-related QOL improver include the lactic acid bacteria cells themselves (also referred to as lactic acid bacterium strains), processed products of lactic acid bacterium strains, and extracts of lactic acid bacterium strains. The component derived from a lactic acid bacterium may be, for example, a component contained in the cell wall of the lactic acid bacterium. Viable cells, wet bacteria, dried bacteria, etc. can appropriately be used as the lactic acid bacterium strain. Examples of the processed product of lactic acid bacteria include lactic acid bacteria, lactic acid bacteria-containing materials, concentrates of fermented milk containing lactic acid bacterium strains, pastes, dried products, liquid products, diluted products, crushed products, etc. The dried product may be at least one selected from the group consisting of spray-dried products, freeze-dried products, vacuum-dried products, and drum-dried products. The processed product of the lactic acid bacterium strain may be a dead bacteria cell. Dead bacteria cells can usually be obtained by heating bacteria cells. The heating conditions are not particularly limited as long as the cells are killed, and in general, sufficient results can be obtained by heating at 105 °C for about 30 minutes. The method of the heat treatment is not particularly limited. Examples of the method for obtaining the processed product of the lactic acid bacterium strain include heat sterilization treatment, radiation sterilization treatment, crushing treatment, etc. The extract of the lactic acid bacterium strain may be extracted from a lactic acid bacterium strain or a processed product thereof with a liquid medium.

The lactic acid bacterium may be of the genus *Lactobacillus,* and may be, for example, *Lactobacillus curvatus,* preferably the *Lactobacillus curvatus* CP2998. The *Lactobacillus curvatus* CP2998 is a type of lactic acid bacteria, which was deposited at the Patent Microorganisms Depositary Center on April 15, 2015 under the accession number: NITE P-02033 and requested to be transferred as NITE BP-20233 to an international deposit based on the Budapest Treaty on April 15, 2019.

A medium for culturing lactic acid bacteria is not particularly limited as long as the lactic acid bacteria can grow in the medium, and can be appropriately selected and used from media generally used for culturing lactic acid bacteria, modified media thereof, etc. A carbon source, a nitrogen source, etc. contained in the medium for culturing lactic acid bacteria are not particularly limited. For example, at least one selected from the group consisting of glucose, sucrose, lactose, molasses, etc. used for culturing ordinary microorganisms can be used as the carbon source. At least one selected from the group consisting of peptone, casein, casein decomposition products, whey, whey decomposition products, etc. can be used as the nitrogen source. For the medium, at least one selected from the group consisting of corn steep liquor (CSL), yeast extract, meat extract, liver extract, tomato juice, etc. may be used as a source of other nutrients. Additionally, a reducing agent such as L-cysteine, a growth promoting factor such as vitamin, nucleic acid-related substance, acetate, citrate, fatty acid ester, or particularly preferably Tween 80, compounds etc. capable of imparting a buffering ability such as phosphate may be added as appropriate to the medium for culturing lactic acid bacteria. Examples of a medium usable for culturing lactic acid bacterium strains include synthetic media such as MRS medium, squeezed juice of vegetables and fruits, and media generally used for producing fermented milk such as milk, soymilk, and reduced skim milk media.

A culturing method for lactic acid bacteria can be performed, for example, by static culturing, neutralization culturing with a constant pH control, etc., and the culturing method is not particularly limited as long as the lactic acid bacteria grow well under the conditions thereof. For example, the lactic acid bacterium strain is cultivated in accordance with a conventional method of culturing lactic acid bacteria and can be obtained from the obtained culture by a bacterium collecting means such as centrifugation.

The form of the walking ability improver is not particularly limited and may be in a form of a medical drug, a quasi-drug, etc. The dosage form may be a formulation for oral administration such as tablets, pills, capsules, granules, powders, troches, syrups, and liquids. These formulations can be manufactured in accordance with known methods using excipients, lubricants, binders, disintegrants, stabilizers, flavoring agents, diluents, coatings, and other additives of any components.

Examples of the excipient include lactose, sucrose, glucose, mannitol, sorbitol, corn starch, potato starch, crystalline cellulose, gum arabic, dextran, dextrin, pullulan, light anhydrous silicic acid, synthetic aluminum silicate, calcium carbonate, calcium hydrogen phosphate, etc. Examples of the lubricant include stearic acid, calcium stearate, magnesium stearate, talc, colloidal silica, etc. Examples of the binder include hydroxypropyl cellulose, hydroxypropyl methyl cellulose, polyvinylpyrrolidone, macrogol, etc. Examples of the disintegrant include low substitution degree hydroxypropyl cellulosic, carboxymethyl cellulose, carboxymethyl cellulose calcium, carboxymethyl starch, sodium carboxymethyl starch, crosslinked polyvinylpyrrolidone, etc. Examples of the stabilizer include methylparaben, propylparaben, benzyl alcohol, etc. The flavoring agent is appropriately selected from, for example, commonly used sweeteners, acidulants, and flavors.

The walking ability improver and the health-related QOL improver may be formed as foods and drinks such as ordinary foods, health foods, health supplements, foods with functional claims, foods for special dietary uses such as foods for specified health use, and nutritionally functional foods. Therefore, this embodiment includes a food or drink for improving a walking ability and a food or drink for improving health-related QOL. The food or drink for improving a walking ability may be a food or drink for improving a walking speed, a food or drink for improving an amount of muscle activity, etc., and the food or drink for improving health-related QOL may be a food or drink for improving "mental health degree", a food or drink for improving "vitality", a food or drink for improving "mental health", etc.

The food or drink may be configured to contain a component derived from a lactic acid bacterium belonging to the genus *Lactobacillus,* and the component may be added to a raw material in a manufacturing process of the food or drink to form the food or drink. Specific examples of the food or drink can include nutritional supplements, soft drinks, fruit juice drinks, dairy products such as milk, processed milk, milk drinks, fermented milk, yogurt, and cheese, processed egg products such as mayonnaise, confectioneries such as jelly, candy, gummi candies, gums, biscuits, crackers, and butter cakes, breads, etc. The component derived from a lactic acid bacterium belonging to the genus *Lactobacillus* may be blended in a livestock feed, or the component may be added to a raw material in a feed manufacturing process to form a feed.

The manufacturing of the food or drink can include appropriately adding other food materials, i.e., various sugars, emulsifiers, thickeners, sweeteners, acidulants, flavors, amino acids, fruit juices, etc. Specifically, at least one selected from the following can appropriately be added: sugars such as sucrose, isomerized sugar, glucose, fructose, palatinose, trehalose, lactose, and xylose; sugar alcohols such as sorbitol, xylitol, erythritol, lactitol, palatinit, reduced sugar syrup, and reduced maltose sugar syrup; high-sweetness sweeteners such as aspartame, stevia, acesulfame potassium, and sucrose; emulsifiers such as sucrose fatty acid ester, glycerin fatty acid ester, and lecithin; thickening (stabilizing) agents such as carrageenan, xanthan gum, guar gum, pectin, and locust bean gum; acidulants such as citric acid, lactic acid, and malic acid; and fruit juices such as lemon fruit juice, orange fruit juice, and berry-based fruit juice. In addition, vitamins such as vitamin A, vitamin B, vitamin C, vitamin D, and vitamin E, and minerals such as calcium, iron, manganese, and zinc may be added.

Regarding a daily intake of the walking ability improver, the health-related QOL improver, the walking ability improving food or drink, the health-related QOL improving food or drink, etc., for example, in the case of an adult, when the component derived from a lactic acid bacterium is converted into the number of bacteria cells, the daily intake can be an amount containing 1×10⁹ or more and 1×10¹³ or less cells, 5×10⁹ or more and 5×10¹² or less cells, or 5×10¹⁰ or more and 3×10¹² or less cells. A content rate of the component derived from a lactic acid bacterium in the walking ability improver etc. is not particularly limited, and may appropriately be adjusted in accordance with ease of manufacturing, a preferable daily intake, etc. Although not particularly limited, the improver and the food or drink are generally orally ingested.

An ingestion period in which at least one of the walking ability and the health-related QOL can be expected to be improved by ingesting the walking ability improver, the health-related QOL improver, etc. is, for example, 1 week or more, 3 weeks or more, or 6 weeks or more.

Regarding the improvement rate of walking ability associated with the ingestion of the walking ability improver, based on the state of non-ingestion, for example, the improvement rate of walking speed may be 3 % or more, 5 % or more, or 8 % or more and may be, for example, 50 % or less. The improvement rate of the cadence may be 1 % or more, 3 % or more, or 4 % or more, and may be, for example, 40 % or less. The improvement rate of stride may be 2 % or more, 5% or more, or 7 % or more, and may be, for example, 50 % or less. The improvement rate of myoelectric potential in the quadriceps femoris may be 10 % or more, 20 % or more, or 30 % or more, and may be, for example, 80 % or less, 60 % or less, or 50 % or less. The improvement rate of walking speed is obtained by dividing a difference between an average value after ingestion and an average value before ingestion by the average value before ingestion, and the same applies to the other improvement rates.

This embodiment includes a method for improving walking ability including administrating a walking ability improver to a subject, and a method for improving health-related QOL including administering a health-related QOL improver to a subject. The subject of administration of the walking ability improver, the health-related QOL improver, etc. is, for example, a mammal, and the mammal may be a human. The subject may also be a non-human animal. The subject of administration of the walking ability improver, the health-related QOL improver, etc. may be a male or female human adult. The walking ability improver, the health-related QOL improver, etc. can be expected to be particularly effective in, for example, human adults in their 50s or older in which walking speed is considered to start decreasing due to aging.

This embodiment includes a use of a component derived from a lactic acid bacterium belonging to the genus *Lactobacillus* in manufacturing of a walking ability improver used for improving walking ability, a use of a component derived from a lactic acid bacterium belonging to the genus *Lactobacillus* in a method for improving walking ability, and a component derived from a lactic acid bacterium belonging to the genus *Lactobacillus* used in the method for improving walking ability. This embodiment also includes a use of a component derived from a lactic acid bacterium belonging to the genus *Lactobacillus* in manufacturing of a health-related QOL improver used for improving health-related QOL, a use of a component derived from a lactic acid bacterium belonging to the genus *Lactobacillus* in a method for improving health-related QOL, and a component derived from a lactic acid bacterium belonging to the genus *Lactobacillus* used in the method for improving health-related QOL.

### Examples

Although the present invention will hereinafter specifically be described with reference to examples, the present invention is not limited to these examples.

### (Test Example 1) Effect of Improving Walking Ability and SF-36 in the Elderly

Method: A capsule containing 5×10¹¹ cells of the *Lactobacillus curvatus* CP2998 and dextrin as the excipient and a placebo capsule containing the same amount of cellulose instead of the *Lactobacillus curvatus* CP2998 were prepared as test agents. The *Lactobacillus curvatus* CP2998 was obtained through liquid culturing using a conventional method, washing by centrifugation, sterilization of a recovered product, and freeze-drying. The subjects were prepared by dividing healthy elderly persons aged 65 to 79 years giving written informed consent into a placebo group (n=65) and a CP2998 group (n=65). The CP2998 group was allowed to take the capsule containing the cells of the CP2998 and the placebo group was allowed to take a placebo capsule once daily for 12 weeks. In week 0, week 6 and week 12, walking speed, cadence, and stride were measured by Walkway (manufactured by ANIMA Corp.). Myoelectric potential measurement (quadriceps femoris) and muscle mass measurement were performed in week 0, week 6 and week 12. SF-36 was performed in week 12. The myoelectric potential was measured by using an electromyogram/evoked potential inspection device (MEB-9402MB manufactured by Nihon Kohden) for the quadriceps femoris during isometric knee extension exercise. The muscle mass was measured by using a body composition analyzer (InBody 470 manufactured by InBody Japan Inc.).

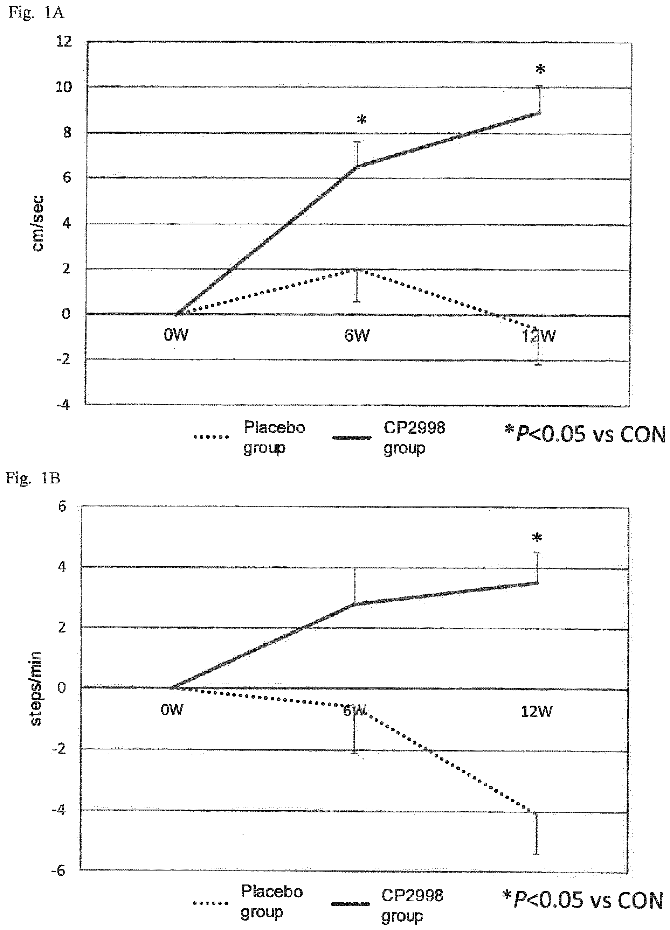

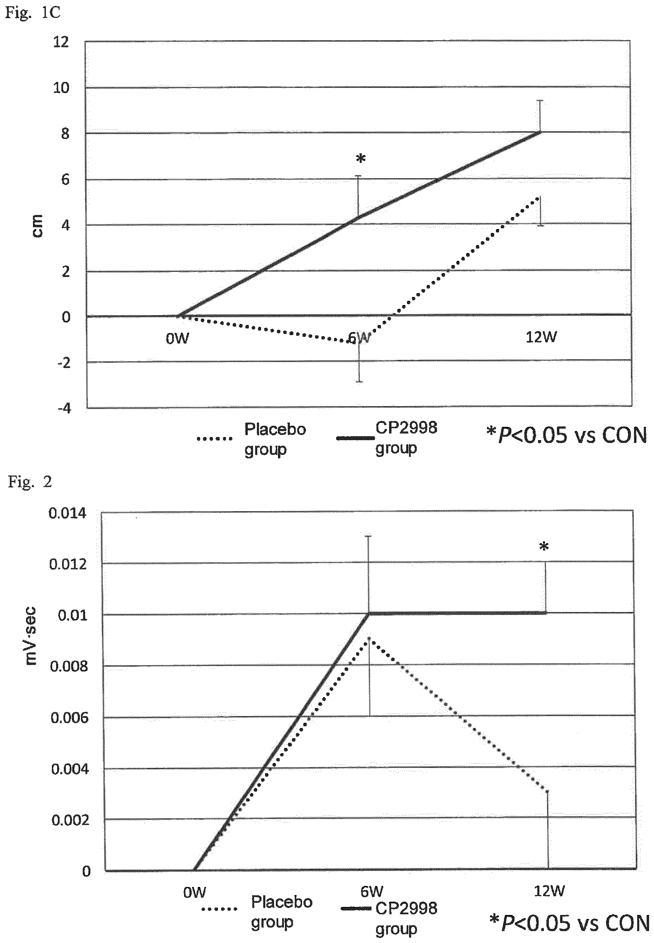

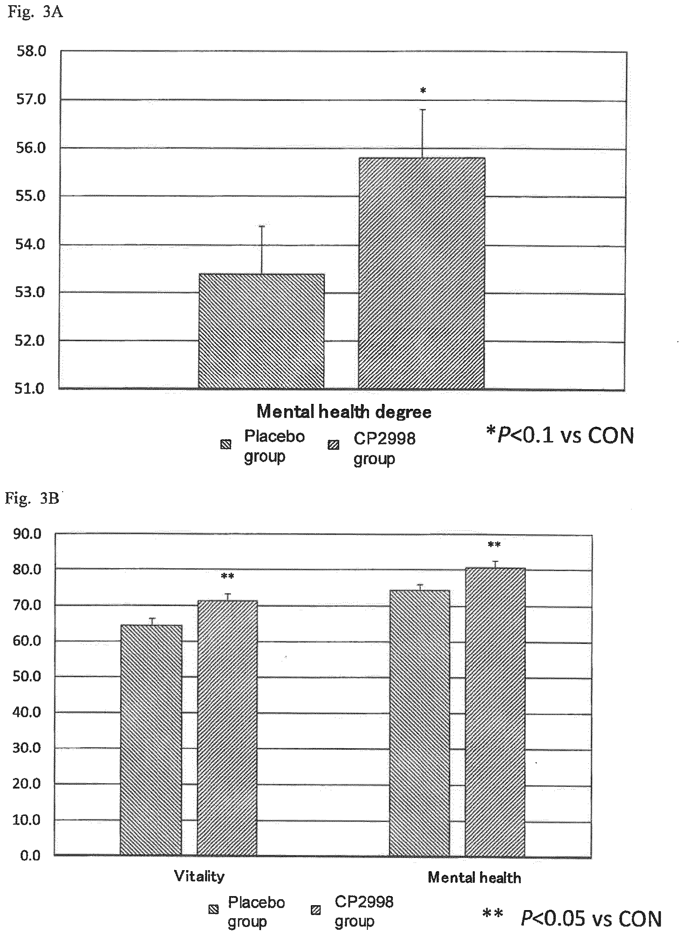

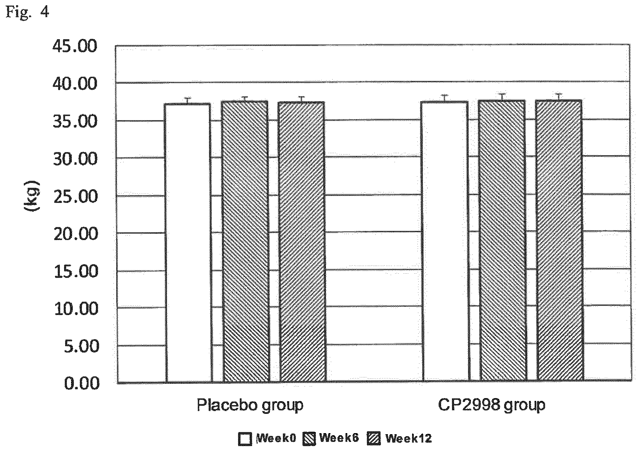

Table 1 shows the measurement results of walking speed, cadence, stride, and myoelectric potential as the transition of walking ability associated with ingestion of the test drug, and respective improvement rates based on week 0. Figs. 1A to 1C respectively show Δwalking speed, Δcadence, and Δstride, which are amounts of change with based on week 0. As the transition of the myoelectric potential, Fig. 2 shows the measurement result of the myoelectric potential as Δ myoelectric potential, which is an amount of change based on week 0. Table 2 shows respective amounts of changes as average values. The results of SF-36 are shown in Figs. 3A and 3B, and the numerical values of the scores are shown in Table 3. Fig. 4 shows the transition of muscle mass in week 0, week 6 and week 12.

**[Table 1]**

| Item | Unit | Group | Week 0 | Week 6 | Improvement rate | Week 12 | Improvement rate |
|---|---|---|---|---|---|---|---|
| Walking speed | cm/sec | Placebo group | 102.9 ± 1.6 | 104.9 ± 1.7 | 1.9% | 102.3 ± 1.5 | -0.6% |
| | | CP2998 group | 1017 ± 1.7 | 108.3 ± 1.6 | 6.5% | 110.6 ± 1.5 | 8.8% |
| Cadence | steps/min | Placebo group | 111.0 ± 1.4 | 110.4 ± 1.4 | -0.5% | 107.0 ± 1.1 | -3.6% |
| | | CP2998 group | 112.4 ± 1.4 | 115.2 ± 1.5 | 2.5% | 115.9 ± 1.2 | 3.1% |
| Stride | cm | Placebo group | 109.6 ± 1.5 | 108.4 ± 1.8 | -1.1% | 114.8 ± 1.3 | 4.7% |
| | | CP2998 group | 106.7 ± 1.7 | 111.0 ± 1.6 | 4.0% | 114.7 ± 1.0 | 7.5% |
| Myoelectric potential | mV·sec | Placebo group | 0.033 ± 0.003 | 0.043 ± 0.003 | 30.3% | 0.036 ± 0.002 | 9.1% |
| | | CP2998 group | 0.029 ± 0.002 | 0.039 ± 0.003 | 34.5% | 0.039 ± 0.003 | 34.5% |

| | | | | | | | |
|---|---|---|---|---|---|---|---|
| Avarage ± Standard Deviation | | | | | | | |

**[Table 2]**

| Item | Unit | Group | 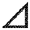 Week 6 | Week 12 |
|---|---|---|---|---|
| Walking speed | cm/sec, 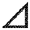 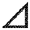 | Placebo group | 2.0 ± 1.4 | -0.6 ± 1.6 |
| | | CP2998 group | 6.5 ± 1.1 * | 8.9 ± 1.2 * |
| Cadence | steps/min 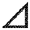 | Placebo group | -0.6 ± 1.5 | -4.1 ± 1.3 |
| | | CP2998 group | 2.8 ± 1.2 | 3.5 ± 1 * |
| Stride | cm | Placebo group | -1.2 ± 1.7 | 5.2 ± 1.3 |
| | | CP2998 group | 4.3 ± 1.8 * | 8.0 ± 1.4 |
| Myoelectric potential | mV·sec | Placebo group | 0.009 ± 0.003 | 0.003 ± 0.002 |
| | | CP2998 group | 0.010 ± 0.003 | 0.010 ± 0.003 * |

| | | | | |
|---|---|---|---|---|
| Avarage ± Standard Deviation * P<0.05 Significant difference compared to placebo group | | | | |

**[Table 3]**

| | Placebo group | CP2998 group | |
|---|---|---|---|
| Physical health degree | 44.5 ± 1.0 | 45.1 ± 1.0 | |
| Mental health degree | 53.4 ± 1.2 | 55.8 ± 1.3 | * |
| Vitality | 64.4 ± 1.9 | 71.2 ± 1.9 | ** |
| Mental health | 74.2 ± 1.7 | 80.8 ± 1.7 | ** |

| | | | |
|---|---|---|---|
| Avarage ± Standard Deviation * P<0.1 Tends to be significant compared to placebo group ** P<0.05 Significant difference compared to placebo group | | | |

As shown in Fig. 1A and Table 1, walking speed was significantly improved in the CP2998 group as compared to the placebo group in week 6 and week 12. As shown in Fig. 1B and Table 1, cadence was significantly improved in the CP2998 group as compared to the placebo group in week 12. As shown in Fig. 1C and Table 1, stride was significantly improved in the CP2998 group as compared to the placebo group in week 6. As shown in Fig. 2 and Table 1, myoelectric potential was significantly improved in the CP2998 group as compared to the placebo group in week 12.

As shown in Fig. 3A, the mental health degree was significantly improved in the CP2998 group as compared to the placebo group. As shown in Fig. 3B, the vitality and the mental health were significantly improved. Improvement in the mental health due to ingestion of the capsules containing the CP2998 means improvement in these mental indexes. The vitality is reduced, for example, due to fatigue. Therefore, this probably means that taking the capsule containing the CP2998 made the subjects less tired, full of vitality, etc. The mental health is scored lower, for example, when feeling melancholy and depressed. Therefore, this probably means that taking the capsule containing the CP2998 improved the depressed mood, and that the subjects were able to spend time healthy and calmly.

Furthermore, as shown in Fig. 4, no intergroup difference was recognized in muscle mass until week 12. Therefore, it can be seen that the walking ability improver improves the walking ability before the difference in muscle mass appears. For example, since the myoelectric potential was improved in week 12, it can be considered that an improvement in nerve response and an improvement in amount of muscle activity increased the walking speed and improved the walking ability.

The disclosures of Japanese Patent Application No. 2019-103777 (Filing Date: June 3, 2019) is hereby incorporated by reference in its entirety. All the documents, patent applications, and technical standards described in this description are hereby incorporated by reference to the same extent as if each of the documents, patent applications, and technical standards is specifically and individually described as being incorporated by reference.

## Claims

1. A walking ability improver comprising a component derived from a lactic acid bacterium belonging to the genus *Lactobacillus.*

2. The improver according to claim 1, wherein the improver improves a walking speed.

3. The improver according to claim 1 or 2, wherein the improver improves an amount of muscle activity.

4. A health-related QOL improver comprising a component derived from a lactic acid bacterium belonging to the genus *Lactobacillus* and improving a mental health degree.

5. The improver according to claim 4, wherein the improver improves vitality.

6. The improver according to claim 4 or 5, wherein the improver improves mental health.

7. The improver according to any one of claims 1 to 6, wherein the lactic acid bacterium is *Lactobacillus curvatus.*

8. A food or drink for improving a walking ability comprising a component derived from a lactic acid bacterium belonging to the genus *Lactobacillus.*

9. The food or drink according to claim 8, wherein the food or drink improves a walking speed.

10. The food or drink according to claim 8 or 9, wherein the food or drink improves an amount of muscle activity.

11. A food or drink for improving health-related QOL, containing a component derived from a lactic acid bacterium belonging to the genus *Lactobacillus* and improving a mental health degree.

12. The food or drink according to claim 11, wherein the food or drink improves vitality.

13. The food or drink according to claim 11 or 12, wherein the food or drink improves mental health.

14. The food or drink according to any one of claims 8 to 13, wherein the lactic acid bacterium is *Lactobacillus curvatus.*
